# EUROPEAN PATENT APPLICATION

(11) **EP 2 384 762 A1**
(43) Date of publication of application: **09.11.2011**
(21) Application number: 10161795.9
(22) Date of filing: 03.05.2010
(51) Int. Cl.: A61K 36/54, A61P 35/00, A61K 36/185

(54) **Use of an extract obtained from plants of the family Canellaceae in the treatment of cancer**

(71) Applicant: Fraunhofer-Gesellschaft zur Förderung der angewandten Forschung e.V., 80686 München (DE)
(72) Inventor: Schubert, Andreas, 04299, Leipzig (DE)
(74) Representative: Maiwald Patentanwalts GmbH

(57) **Abstract**

The invention relates to alcoholic and organic extracts of plant of the genus *Warburgia* for the treatment of cancers and other diseases.

## Description

### FIELD OF THE INVENTION

The present invention pertains to plant extracts obtained from plants of the family *Canellaceae* and preferably of the genus *Warburgia* which can be used in the treatment of diseases caused by microorganisms and/or cancers. The present invention further pertains to pharmaceutical compositions comprising such extracts as well as methods making use of such extracts.

### BACKGROUND OF THE INVENTION

It has been increasingly recognised over the past years that the plant kingdom is one of the major reservoirs for therapeutically active agents.

To date, the complexity of the components contained within plants is not fully understood and it is appreciated that an in-depth biochemical analysis of the components of plants as they are found e.g. in leaves, may reveal valuable therapeutic compounds for the treatment of various diseases as diverse as microbial infections or cancer.

Further, it has been recognised that extracts of plants may be valuable both as a source allowing for further identification of active compounds contained therein and as a pharmaceutical composition in itself.

Thus, there is a continuing need for plant extracts and pharmaceutical compositions comprising such plant extracts being useful for the treatment of diseases.

### OBJECTIVE AND SUMMARY OF THE INVENTION

It is one objective of the present invention to provide plant extracts which can be used for the treatment of diseases as they are caused by infections with microorganisms or diseases such as cancer.

It is a further objective of the present invention to provide pharmaceutical preparations which can be used in the treatment of diseases caused by infections with microorganisms or of diseases such as cancer.

Yet another objective of the present invention relates to the provision of methods allowing for treatment of individuals suffering from diseases caused by infections with microorganisms or from diseases such as cancer.

These and other objectives as they become apparent from the ensuing description are attained by the subject matter of the independent claims. Some of the preferred embodiments are set out in the dependent claims.

One aspect of the present invention relates to an extract obtainable from plants wherein said plants are selected from the family *Canellaceae.* In a preferred embodiment of this aspect of the invention, the extracts are obtainable from plant of the *Warburgia* genus. Yet a further preferred embodiment of this aspect of the invention relates to plant extracts obtainable from *Warburgia* species belonging to the group comprising:
*Warburgia salutaris,*
*Warburgia ugandensis,*
*Warburgia stuhlmannii,*
*Warburgia elongata,* and
*Warburgia breyeri.*

A particularly preferred *Warburgia* species in the context of the present invention is *Warburgia salutaris* or *Warburgia ugandenis.*

Extracts as described hereinafter are obtainable by subjecting material obtained from fruits, roots, leaves and/or bark of *Canellaceae* such as e.g. the aforementioned *Warburgia* species to preferably organic, aqueous and/or alcoholic extraction liquids. Preferably the material comes from the bark of *Canellaceae* such as e.g. the aforementioned *Warburgia* species, with *Warburgia salutaris* or *Warburgia ugandenis* being particularly preferred.

The present invention in another embodiment relates to a pharmaceutical composition comprising extracts as mentioned hereinafter.

Such pharmaceutical compositions may be used for the treatment of diseases caused by infections with microorganisms (e.g. microbial infections) and/or for the treatment of cancer.

Thus, pharmaceutical compositions may be used for the treatment of diseases caused by the phylae of bacteria or caused by eukaryotic microorganisms.

Further, pharmaceutical compositions in accordance with the invention can be used for the treatment of cancers such as lung cancer (including non small cell lung cancer), kidney cancer, bowel cancer, head and neck cancer, colo(rectal) cancer, glioblastom, breast cancer, prostate cancer, skin cancer, melanoma, non Hodgkin lymphoma and the like.

In particular, pharmaceutical compositions in accordance with the invention can be used for the treatment of cancers they are defined according to the International Classification of Diseases in the field of oncology (see http://en.wikipedia.org/wiki/carcinoma). Such cancers include epithelial carcinomas such as epithelial neoplasms; squamous cell neoplasms including squamous cell carcinoma; basal cell neoplasms including basal cell carcinoma; transitional cell papillomas and carcinomas; adenomas and adenocarcinomas (glands) including adenoma, adenocarcinoma, linitis plastic, insulinoma, glucagonoma, gastrinoma, vipoma, cholangiocarcinoma, hepatocellular carcinoma, adenoid cystic carcinoma, carcinoid tumor, prolactinoma, oncocytoma, hurthle cell adenoma, renal cell carcinoma, grawitz tumor, multiple endocrine adenomas, endometrioid adenoma; adnexal and skin appendage Neoplasms; mucoepidermoid neoplasms; cystic, mucinous and serous neoplasms including cystadenoma, pseudomyxoma peritonei; ductal, lobular and medullary neoplasms; acinar cell neoplasms; complex epithelial neoplasms including Warthin's tumor, thymoma; specialized gonadal neoplasms including sex cord-stromal tumor, thecoma, granulosa cell tumor, arrhenoblastoma, Sertoli-Leydig cell tumor; paragangliomas and glomus tumors including paraganglioma, pheochromocytoma, glomus tumor; nevi and melanomas including melanocytic nevus, malignant melanoma, melanoma, nodular melanoma, dysplastic nevus, lentigo maligna melanoma, superficial spreading melanoma and acral lentiginous malignant melanoma.

The use of pharmaceutical compositions in accordance with the invention for the treatment of lung cancer, breast cancer, kidney caner and in particular glioblastom is particularly preferred.

The present invention further relates to the use of extracts as defined hereinafter in the manufacture of a medicament for the treatment of diseases caused by microbial infections and/or cancer. Such medicaments may thus be used for the treatment of diseases caused by the phylae of bacteria or for the treatment of diseases caused by eukaryotic microorganisms such as fungi or *Trypanosoma.* Further, such medicaments may be used in the treatment of the aforementioned cancers.

Yet another embodiment of the present invention relates to methods of treating humans or non-humans suffering from microbial infections and/or cancer by administering extracts or pharmaceutical compositions as defined above to said individuals.

### FIGURE LEGENDS

- **Figure 1:**: Figure 1 displays the effects of aqueous extracts on the cell line TD47.
- **Figure 2:**: Figure 2 displays the effects of organic extracts on the cell line TD47.
- **Figure 3:**: Figure 3 displays the effects of aqueous extracts on the cell line HT29
- **Figure 4:**: Figure 4 displays the effects of organic extracts on the cell line HT29:
- **Figure 5:**: Figure 5 displays the effects of aqueous extracts on the cell line CRL-1573.
- **Figure 6:**: Figure 6 displays the effects of organic extracts on the cell line CRL-1573.
- **Figure 7:**: Figure 7 displays the effects of alcoholic extracts on the cell line CRL-1573.
- **Figure 8:**: Figure 8 displays the effects of aqueous extracts on the cell line HT-11.
- **Figure 9:**: Figure 9 displays the effects of organic extracts on the cell line HT-11.
- **Figure 10:**: Figure 10 displays the effects of aqueous extracts on the cell line HUVEC.
- **Figure 11:**: Figure 11 displays the effects of organic extracts on the cell line HUVEC.

### DETAILED DESCRIPTION OF THE INVENTION

Although the present invention will be described with respect to particular embodiments, this description is not to be construed in a limiting sense.

Before describing in detail exemplary embodiments of the present invention, definitions important for understanding the present invention are given. It is to be understood that unless defined otherwise, all technical and scientific terms used herein have the same meanings as commonly understood by one of ordinary skill in the art.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

The term "obtainable" also includes as a preferred embodiment the term "obtained". Thus, if e.g. an abstract is stated to be "obtainable" by e.g. alcoholic extraction of e.g. *Warburgia ugandensis,* as a preferred embodiment this includes an extract which is obtained by alcoholic extraction of *Warburgia ugandensis.*

Further definitions of terms may be provided in the context in which the terms are introduced.

The present invention relates in one embodiment to an extract obtainable from plants which are selected from the *Canellaceae* family. Preferably, these extracts are obtained from the *Warburgia* genus. Preferred *Warburgia* species from which extracts are obtainable comprise:
*Warburgia salutaris,*
*Warburgia ugandensis,*
*Warburgia stuhlmannii,*
*Warburgia elongata,* and
*Warburgia breyeri.*

A particularly preferred *Warburgia* species in accordance with the present invention is *Warburgia ugandensis* or *Warburgia salutaris.*

Plant extracts obtainable from the aforementioned *Canellaceae* family and preferably from the *Warburgia* species and in particular from *Warburgia ugandensis* or *Warburgia salutaris* can be used for the treatment of microbial infections and/or cancer by administering pharmaceutically effective amounts of such extracts to a patient suffering from such diseases and/or for the identification of pharmaceutically active compounds contained within such extracts.

Extracts in accordance with the invention are obtainable by subjecting material obtained from fruits, roots, leaves, bark and/or other parts of the *Canellaceae* family and preferably of the abovementioned *Warburgia* species to extraction liquids. Preferably, the material to be extracted is from the bark of *Warburgia* species such as *Warburgia ugandensis* or *Warburgia salutaris.*

For the extraction of material of *Warburgia* species such as *Warburgia* ugandensis one may use aqueous, organic and/or alcoholic extraction liquids. The use of aqueous and/or organic extraction liquids can be preferred.

An example for an aqueous extraction liquid is water.

Typically, alcoholic extraction liquids comprise halogenated or non-halogenated, primary, secondary or tertiary, branched or non-branched alcohols having a chain with C₁-C₁₀ carbon atoms. These alcohols may be mixed with water in different ratios. Particularly preferred are methanol, ethanol, propanol or butanol, and their respective mixtures with water such as 70% (v/v) ethanol.

It is understood by the skilled person that the alcohol component may be present in the alcoholic extraction liquid in different amounts. Typically, the alcohol component is present in an amount in the range of about 50% (v/v) to about 98% (v/v), preferably in the range of about 60% (v/v) to about 95% (v/v) and more preferably in the range of about 65% (v/v) to about 95% (v/v) such as in an amount of about 70% (v/v), in an amount of about 75%, in an amount of about 80% (v/v), in an amount of about 85% (v/v) or in an amount of about 90% (v/v) based on the complete volume of the extraction liquid.

If an alcoholic extraction liquid with the above mentioned alcohol amounts is used, the remaining parts of this liquid may be made up by other components with water being the preferred component.

One of the most preferred extraction liquids comprises ethanol as a first alcoholic component in the range of about 60% (v/v) to about 80% (v/v) such as about 70% (v/v) ethanol and water as a second non-alcoholic component.

Thus, the present invention in a particularly preferred aspect relates to extracts obtained from of the *Canellaceae* family and preferably from the abovementioned *Warburgia* species by subjecting material such as bark to an alcoholic extraction liquid which comprises ethanol as a first alcoholic component in the range of about 60% (v/v) to about 80% (v/v) such as about 70% (v/v) and water as a second non-alcoholic component. Extracts obtained from bark of *Warburgia ugandensis* by such a liquid are most preferred in accordance with the invention.

Another embodiment of the present invention relates to extracts which are obtainable by organic extraction liquids. Organic extraction liquids can include halogenated or non-halogenated, branched or non-branched hydrocarbons having a chain with C₁ to C₁₀ carbon atoms. Such organic extraction liquids may comprise CH₂Cl₂, CHCl₃, toluol, tetrahydrofuran, diethylether, and apolar solvents such as n-alkanes including petroleum ether.

Extracts may be obtainable from the *Canellaceae* family and preferably of the abovementioned *Warburgia* species such as *Warburgia ugandensis* by subjecting material such as bark to alcoholic and/or organic extraction liquids at elevated temperatures for prolonged periods of time.

If an alcoholic extraction liquid comprising ethanol as a first alcoholic component in the range of about 60% (v/v) to about 80% (v/v) such as about 70% (v/v) ethanol and water as a second non-alcoholic component is used for obtaining an extract from e.g. bark material of e.g. *Warburgia ugandensis,* extraction may be undertaken by subjecting the material for e.g. about 10 to about 48 hours, such as about 12 to about 36 hours or about 24 hours to the extraction liquid at room temperature or at elevated temperatures such as up to 30°C, up to 40°C or up to 50°C.

If organic extraction liquids are used, such as CH₂Cl₂, material such as bark material of e.g. *Warburgia ugandensis* may be subjected to the organic extraction liquid for about 4 to about 48 hours, such as about 8 to about 24hous at room temperature or at elevated temperatures such as up to 30°C, up to 40°C or up to 50°C.

The extracted material may be separated from the starting material by simply centrifuging the extraction reaction. This will cause extracted material such as bark powder, leaf tissue etc., to sediment so that the supernatant comprising the respective pharmaceutically active components can be taken off. In order to further purify the supernatants, they may e.g. be filtrated. After filtration, the alcoholic and/or organic extracts may either be directly used or further be reduced by e.g. vaporisation. The residual material may then be solubilized in suitable liquids, including alcoholic solutions such as 70% (v/v) ethanol and organic solvents. However, the extracts may also be diluted into other liquids such as 70% (v/v) ethanol, water and/or organic solvents

The extracts may be obtained by simply boiling the material such as bark or leave powders in the respective extraction liquid. Thereafter, the extracts are separated from the material by the afore-mentioned methods.

In case of aqueous extraction liquids such as water, bark or leaf powder may be boiled in about 50 to about 1000 ml of the extraction liquid for about 1 to about 24 h. Typical extraction conditions include e.g. boiling the material in about 50 to about 200 ml of water such as about 100 ml for about 1 to about 3h such as about 2h.

In case of organic extraction liquids such as CH₂Cl₂, bark or leaf powder may be boiled in about 50 to about 1000 ml of the extraction liquid for about 1 to about 24 h. Typical extraction conditions include e.g. boiling the material in about 50 to about 200 ml of CH₂Cl₂ such as about 100 ml for about 7 to about 9h such as about 8h.

In case of alcoholic extraction liquids such as 70% (v/v) ethanol, bark or leaf powder may be boiled in about 50 to about 1000 ml of the extraction liquid for about 1 to about 24 h. Typical extraction conditions include e.g. boiling the material in about 50 to about 200 ml of 70% (v/v) ethanol such as about 100 ml for about 20 to about 24h such as about 8h.

As mentioned above, the extracts in accordance with the present invention may be used to further identify pharmaceutically active components contained within said extracts.

Alternatively and/or in addition, the extracts may be formulated into pharmaceutical compositions by e.g. combining pharmaceutically active amounts of such extracts optionally together with pharmaceutically acceptable excipients.

In one embodiment the present invention therefore relates to a pharmaceutical composition comprising an extract obtained from a plant of the *Canellaceae* family and preferably of plants of the *Warburgia* genus. Preferably, pharmaceutical compositions in accordance with the invention comprise an extract obtained from a *Warburgia* species being selected from the group comprising:
*Warburgia salutaris,*
*Warburgia ugandensis,*
*Warburgia stuhlmannii,*
*Warburgia elongata,* and
*Warburgia breyeri.*

Such pharmaceutical compositions can be used for the treatment of microbial infections and preferably for the treatment of cancer. More preferably such pharmaceutical compositions, particularly if they are used in the treatment of cancers, comprise an extract obtained by organic, aqueous or alcoholic extraction.

If aqueous extraction is used, one may use water as described above

If alcoholic extraction is used, one may use the alcoholic extraction liquids as described above, with an extraction liquid comprising ethanol as a first alcoholic component in the range of about 60% (v/v) to about 80% (v/v) such as about 70% (v/v) ethanol and water as a second non-alcoholic component being preferred. As regards organic extraction, one may use organic extraction liquids as described above, with the organic extraction liquid CH₂Cl₂ being preferred.

If the extracts are dried and dissolved as described above, the extracts may comprise about 50 ng to 100 ng dry extract , preferably about 10 ng to about 50 ng dry extract and more preferably about 15 ng to about 30 ng of dry extract per ml administration liquid.

If the extracts are diluted as described above, the extracts may have a concentration in the dilution liquid of about 1:10: to about 1:500 such as about 1:50, about 1:100, about 1:200, about 1:250, about 1:300, about 1: 350, about 1:400, about 1:450, about 1:500, about 1:550, about 1:600, about 1:650, about 1:700, about 1:750, about 1:800 or about 1:900. The dilution liquid may be e.g. 70% (v/v) ethanol.

Most preferably, pharmaceutical compositions in accordance with the invention suitable for the treatment of cancer comprise a pharmaceutically effective amount of an extract obtained from bark material of *Warburgia ugandensis* or *Warburgia salutaris* using an extraction liquid comprising ethanol as a first alcoholic component in the range of about 60% (v/v) to about 80% (v/v) such about 70% (v/v) ethanol and water as a second non-alcoholic component, using water as an extraction liquid or using CH₂Cl₂ as an organic extraction liquid.

As mentioned above, extracts and pharmaceutical compositions in accordance with this invention may be used for the treatment of microbial infections as they are caused by microorganisms.

The person skilled in the art will therefore understand that the term "pharmaceutical composition" indicates that the composition contains a pharmaceutically effective amount of an extract in accordance with the invention. Thus, the term "effective amount" as used herein is intended to mean an amount of extract in accordance with the invention that is sufficient to display the activity required to treat microbial infections and/or cancer.

The term "microorganism" is used as known in the art and refers to prokaryotic as well eukaryotic microorganisms.

Thus, extracts and pharmaceutical compositions in accordance with the invention may display an anti-microbial activity against the different phylae of bacteria. These phylae comprise proteo bacteria, gram-positive bacteria, cyano bacteria and prochlorophytes, chlamydia, planctomyces/perellula, verrucomicrobia, flavobacteria, cytophaga, green sulphur bacteria, spirochetes, deinococci, green non-sulphur bacteria, deeply branching hyperthermophyllic bacteria and nitrospira/defferibacter. The peptides in accordance with the invention may also be used against the phylae of archae including the phylae euryarchaeota and crenarchaeota.

As regards eukaryotic microorganisms, extracts and pharmaceutical compositions in accordance with the invention may be used against protozoa, fungi, slime molds and algae.

Diseases resulting from microbial infections include airborne transmitted diseases, diseases that are transmitted by person-to-person contact as well as sexually transmitted diseases.

Examples of the aforementioned types of airborne diseases that result from microbial infections are e.g. streptococcal diseases, induced by streptococci, diphtheria, which is induced by *Corynebacterium diptheriae,* whooping cough, which is a consequence *of Bordetella pertussis,* tuberculosis resulting from *Mycobacterium tuberculosis,* meningitis resulting from *Neissereria meningitides.*

Examples of diseases resulting from person-to-person contact are diseases provoked by *staphylococci,* gastric ulcers resulting *Helicobacter pylori.* Examples of sexually transmitted diseases being a consequence of microbial infection include e.g. gonorrhea and syphilis.

The pharmaceutical compositions in accordance with the invention may also be used to treat diseases other than those mentioned previously, including animal-transmitted diseases, arthropod-transmitted diseases and soil-borne diseases. Examples of the aforementioned diseases include Lyme disease, malaria, Rickettsial disease.

Other diseases in the context of the present invention include water-borne microbial diseases such as cholera, giardiasis, legionellosis and typhoid fever as well as food-borne diseases such as staphylococcal food poisoning, clostridial food poisoning, salmonellosis and listeriosis.

A particular focus of the present invention relates to pharmaceutical compositions, which can be used to treat diseases resulting from infective fungi.

Examples of infective fungi induced diseases are superficial mycosis (dermatomycosis), subcutaneous mycosis and systemic mycosis.

Examples of superficial mycosis include ringworm, favus, athlete's foot and jock itch. In the context of these diseases, the peptides in accordance with the invention can act e.g. against microsporum, trichophyton and epidermophyton.

Examples of subcutaneous mycoses are sporotrichosis resulting from *Sporothrix schenckii* or chromoblastomycoses.

Systemic mycoses include cryptococcosis resulting from *Cryptococcus neofurmans,* coccidioidomycosis resulting from *Coccidioidis immitis,* histoplasmosis resulting from *Histoplasma capsulatum,* blastomycosis resulting from *Blastomyces dermatitidis,* candidiasis resulting from *Candida albicans.* These infections will typically be treated using pharmaceutical compositions.

Other disease which can be treated with extracts and pharmaceutical compositions in accordance with the invention are caused by microorganisms such as *Streptococcus mutans, Staphylococcus aureus, Klebsiella sp., Enterococcus spec., Candida albicans* or *Trypanosoma brucei.*

In a particularly prefrerred embodiment, extracts and pharmaceutical compositions in accordance are used for the treatment of cancer.

The term "cancer" in the context of the present invention relates to lung cancer, kidney cancer, head and neck cancer, bowel cancer, colon cancer, glioblastom, breast cancer, prostate cancer, skin cancer, melanoma, lymphoma. Other cancers are mentioned above.

Most preferably, pharmaceutical compositions in accordance with the invention are used for cancers such as breast cancer, lung cancer, kidney cancer and even more preferably for glioblastoma.

Compositions comprising an extract obtained by extraction of bark material of e.g. *Warburgia ugandensis* with the above mentioned aqueous, alcoholic or organic extraction liquid comprising e.g. water or e.g. ethanol as a first alcoholic component in the range of about 60% (v/v) to about 80% (v/v) such as about 70% (v/v) and water as a second non-alcoholic component or e.g. CH₂Cl₂ are particularly suitable for the treatment of e.g. breast cancer, colon cancer, kidney cancer and glioblastoma given that the experiments herein indicate that the cell lines TD47, HT29,CRL-1573 and HT-11 are more sensitive to such extracts than e.g. normal epithelial cells represented by cell line such as HUVEC.

Extracts and pharmaceutical compositions in accordance with the invention may be administered orally. Extracts and pharmaceutical compositions in accordance with the invention may also be administered by any other convenient route, for example, by infusion or bolus injection, by absorption through epithelial or mucocutaneous linings (e.g., oral, rectal, and intestinal mucosa, etc.) and may be administered together with a second therapeutically active agent. Administration can be systemic or local. Various delivery systems are known, e.g., encapsulation in liposomes, microparticles, microcapsules, multiparticulates, capsules, etc., and can be used to administer extracts and pharmaceutical compositions in accordance with the invention.

Methods of administration include, but are not limited to, intradermal, intramuscular, intraperitoneal, parenteral, intravenous, subcutaneous, intranasal, epidural, oral, sublingual, intracerebral, intravaginal, transdermal, rectal, by inhalation, or topical, particularly to the ears, nose, eyes, or skin.

Pharmaceutical dosage forms may be solid or liquid dosage forms or may have an intermediate, e.g. gel-like character depending *inter alia* on the route of administration.

The dosage forms may comprise various pharmaceutically acceptable excipients which will be selected depending on which functionality is to be achieved for the dosage form. For example the pharmaceutical compositions may be delayed and/or prolonged dosage forms.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants. The term carrier denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

Typical pharmaceutically acceptable excipients include substances like sucrose, manitol, sorbitol, starch and starch derivatives, lactose, and lubricating agents such as magnesium stearate, disintegrants and buffering agents.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents.

Further conventional excipients, which can be used in the aforementioned dosage forms depending on the functionality that is to be achieved for the dosage form, include pharmaceutically acceptable organic or inorganic carrier substances which do not react with the active compound. Suitable pharmaceutically acceptable carriers include, for instance, water, salt solutions, alcohols, oils, preferably vegetable oils, polyethylene glycols, gelatin, lactose, amylose, magnesium stearate, surfactants, perfume oil, fatty acid monoglycerides and diglycerides, petroethral fatty acid esters, hydroxymethyl-cellulose, polyvinylpyrrolidone and the like. The pharmaceutical preparations can be sterilized and if desired, mixed with auxiliary agents, like lubricants, preservatives, stabilizers, wetting agents, emulsifiers, salts for influencing osmotic pressure, buffers, colorings, flavoring and/or aromatic substances and the like which do not deleteriously react with the active compounds. For parenteral application, particularly suitable vehicles consist of solutions, preferably oily or aqueous solutions, as well as suspensions, emulsions, or implants.
Some embodiments of the invention thus relate to :
1. A pharmaceutical composition comprising an extract obtained from a plant of the *Canellaceae* family, preferably from a species of the *Warburgia* genus for use in the treatment of cancer.
2. An extract obtained from plants wherein said plants are selected from the *Canellaceae* family.
3. An extract according to 2., wherein the plants is a species of the *Warburgia* genus.
4. An extract of 3., wherein said *Warburgia* species is selected from the group comprising:
   *Warburgia salutaris,*
   *Warburgia ugandensis,*
   *Warburgia stuhlmannii,*
   *Warburgia elongata,* and
   *Warburgia breyeri.*
5. An extract of 4., wherein said *Warburgia* species is *Warburgia ugandensis.*
6. An extract of any of 2. to 5., wherein said extract is obtainable by subjecting material obtained from fruits, roots, leaves and/or bark of *Warburgia* species to organic, aqueous or alcoholic extraction liquids.
7. An extract of 6., wherein said material is from bark of *Warburgia* species.
8. An extract of 6. or 7., wherein said aqueous extraction liquid is water.
9. An extract of 6. or 7., wherein said alcoholic extraction liquid comprises methanol, ethanol, propanol, butanol as the alcohol component.
10. An extract of 9., wherein the alcohol component is present in an amount in the range of about 50% (v/v) to about 98% (v/v), preferably in the range of about 60% (v/v) to about 95% (v/v) and more preferably in the range of about 65% (v/v) to about 95% (v/v) such as in an amount of about 70% (v/v), in an amount of about 75%, in an amount of about 80% (v/v), in an amount of about 85% (v/v) or in an amount of about 90% (v/v) based on the complete volume of the extraction liquid.
11. An extract of 10., wherein the extraction liquid comprises water as the component different from the alcoholic component.
12. An extract of 11., wherein the extraction liquid comprises ethanol as a first alcoholic component in the range of about 60% (v/v) to about 80% (v/v) such about 70% (v/v) ethanol and water as a second non-alcoholic component.
13. An extract of any of 2. to 12., wherein said extract is obtained by subjecting bark material of *Warburgia* species to an extraction liquid comprising ethanol as a first alcoholic component in the range of about 60% (v/v) to about 80% (v/v) such about 70% (v/v) ethanol and water as a second non-alcoholic component.
14. An extract of 13., wherein said *Warburgia* species is *Warburgia ugandensis* and wherein said extract is obtained by subjecting bark material of *Warburgia ugandensis* to an extraction liquid comprising ethanol as a first alcoholic component in the range of about 60% (v/v) to about 80% (v/v) such about 70% (v/v) ethanol and water as a second non-alcoholic component.
15. An extract of 6. or 7., wherein said organic extraction liquid comprises CH₂Cl₂, CHCl3, toluol, tetrahydrofuran, diethylether or petroleum ether.
16. An extract of any of 15., wherein said extract is obtained by subjecting bark material of *Warburgia* species to an extraction liquid comprising CH₂Cl₂.
17. An extract of 16., wherein said *Warburgia* species is *Warburgia ugandensis* and wherein said extract is obtained by subjecting bark material of *Warburgia ugandensis* to an extraction liquid comprising CH₂Cl₂.
18. Pharmaceutical composition comprising an extract of any of 2. to 17. and optionally pharmaceutically acceptable excipients.
19. Pharmaceutical composition comprising an extract of any of 2. to 17. and optionally pharmaceutically acceptable excipients for use in the treatment of diseases caused by microbial infections and/or cancer.
20. Pharmaceutical composition of 19. for use in the treatment of diseases caused by the phylae of bacteria.
21. Pharmcaeutical composition of 19. for use in the treatment of diseases caused by eukaryotic microorganisms.
22. Pharmaceutical composition of 21. for use in the treatment of diseases caused by fungi.
23. Pharmaceutical composition of 21. for use in the treatment of infections caused by *Trypanosoma,* preferably for the treatment of infections caused by *Trypanosoma brucei.*
24. Pharmaceutical composition of 19. for use in the treatment of cancer wherein said cancer is selected from the group comprising lung cancer, kidney cancer, head and neck cancer, colon cancer, bowel cancer, glioblastom, breast cancer, prostate cancer skin cancer, or melanoma.
25. Pharmaceutical composition of 24. for use in the treatment of breast cancer, colon cancer, kidney cancer and/or glioblastoma.
26. Use of an extract as defined in any of 2. to 17. in the manufacture of a medicament for the treatment of diseases caused by microbial infections and/or cancer.
27. Use of 26. for the treatment of diseases caused by the phylae of bacteria.
28. Use of 26. for the treatment of diseases caused by eukaryotic microorganisms.
29. Use of 28. for the treatment of diseases caused by fungi.
30. Use of 28. for the treatment of infections caused by *Trypanosoma,* preferably for the treatment of infections caused by *Trypanosoma brucei.*
31. Use of claim 26. for the treatment of cancer wherein said cancer is selected from the group comprising lung cancer, kidney cancer, head and neck cancer, colon cancer, bowel cancer, glioblastom, breast cancer, prostate cancer, skin cancer, or melanoma.
32. Use of claim 26. for the treatment of cancer wherein said cancer is selected from breast cancer, colon cancer, kidney cancer and/or glioblastoma.
33. Method of treating a human or non-human individual suffering from microbial infections and/or cancer by administering an extract as defined in any of 1. to 17. to said individual.

### EXAMPLES

### Example 1 - Preparing aqueous extracts of Warburgia ugandensis

Bark material of *Walburgia ugandensis* was obtained. 10 g of bark powder was boiled for 2 h in 100 ml water followed by centrifugation and sterile filtration of the supernatant.

### Example 2 - Preparing alcoholic extracts of Warburgia ugandensis

Bark material of *Walburgia ugandensis* was obtained. 10 g of bark powder was boiled for 24 h in 100 ml 70% (v/v) Ethanol followed by centrifugation and sterile filtration of the supernatant.

### Example 3 - Preparing organic extracts of Warburgia ugandensis

Bark material of *Warburgia ugandensis* was obtained. 10g of bark powder was boiled for 8 h in 100 ml CH₂Cl₂ in a Soxlhet apparture followed by centrifugation. Subsequently the supernatant was dried and resuspended in 100 ml of 70% (v/v= ethanol.

### Example 4 - Effects of extracts on the breast cancer cell line T47D

The breast cancer cell line T47D was cultivated at 37°C, 95% humidity, 5% CO₂ for different times in standard medium comprising the aqueous extract (AE) of Example 1 at dilutions of 1:500, 1:333 and 1:250. As control, either pure cell culture medium (ctrl.) or cell culture medium with 70% alcohol at a 1:250 dilution (e-ctrl.) was used.

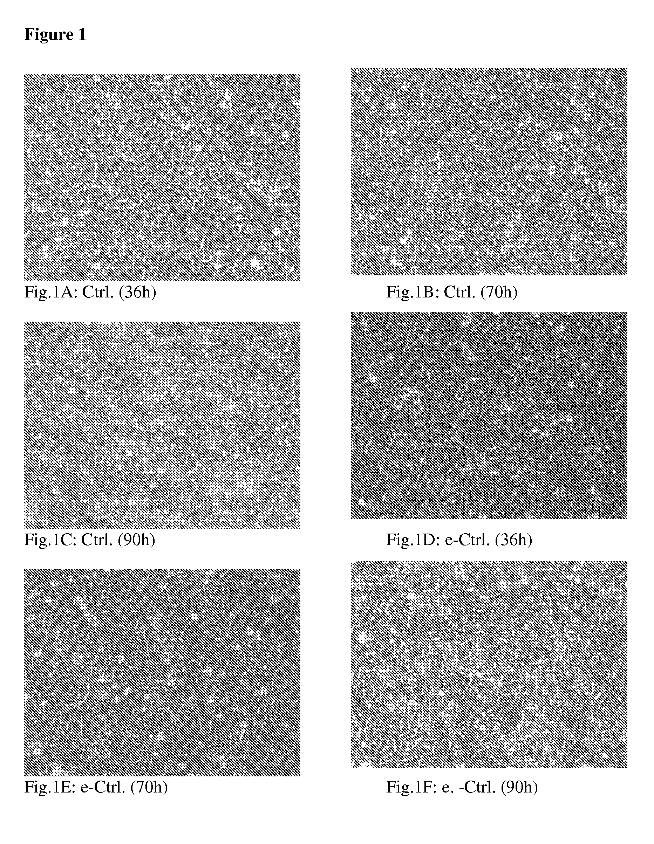

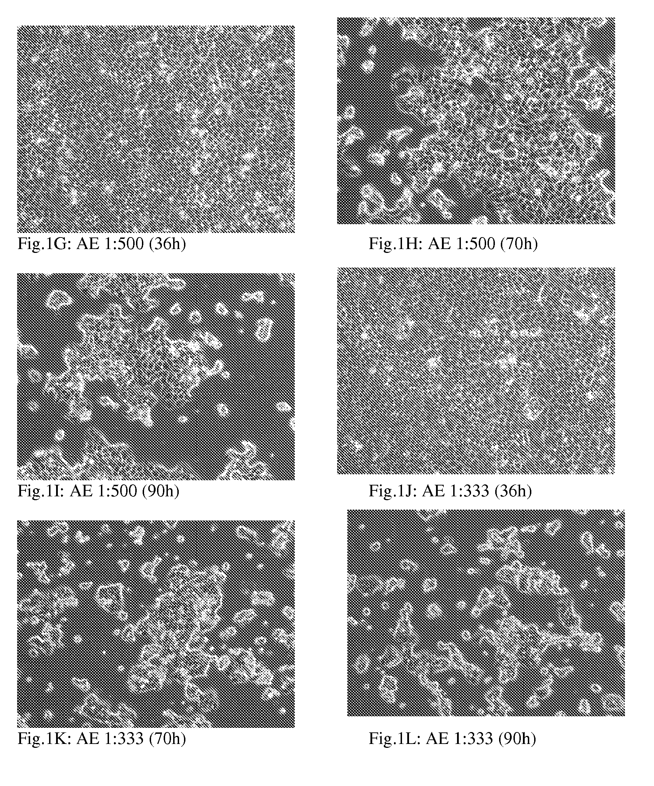

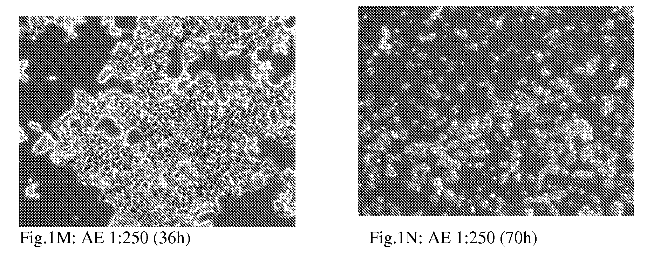

The results (see Figures 1A to 1N) show that with increasing amounts of the aqueous extract, cells display a decreasing vitality and ultimately die.

The breast cancer cell line T47D was cultivated at 37°C, 95% humidity, 5% CO₂ for different times in standard medium comprising the organic extract (OE) of Example 3 at dilutions of 1:500, 1:333 and 1:250. As control, either pure cell culture medium (ctrl.) or cell culture medium with 70% alcohol at a 1:250 dilution was used.

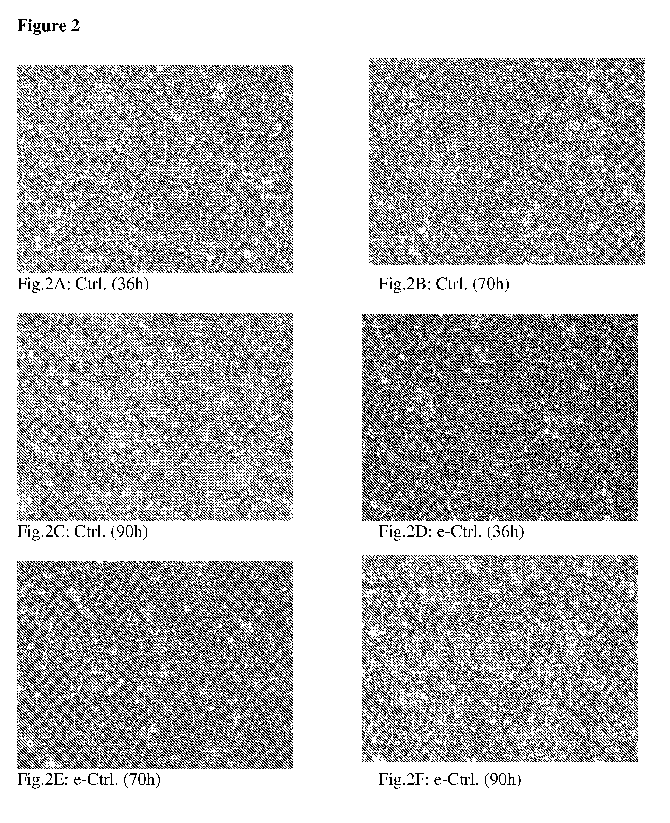

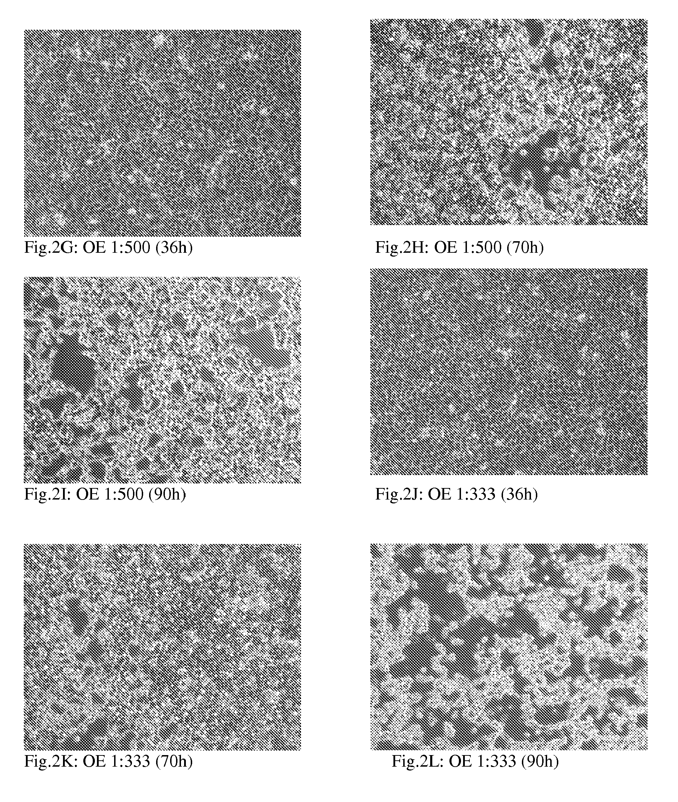

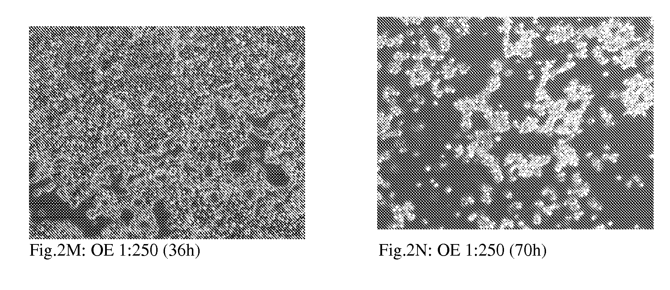

The results (see Figures 2A to 2N) show that with increasing amounts of the organic extract, cells display a decreasing vitality and ultimately die.

### Example 5 - Effects of extracts on the colon cancer cell line HT29

The colon cancer cell line HT229 was cultivated at 37°C, 95% humidity, 5% CO₂ for different times in standard medium comprising the aqueous extract (AE) of Example 1 at dilutions of 1:333 and 1:250. As control, either pure cell culture medium (ctrl.) or cell culture medium with 70% alcohol at a 1:250 dilution (e-ctrl.) was used.

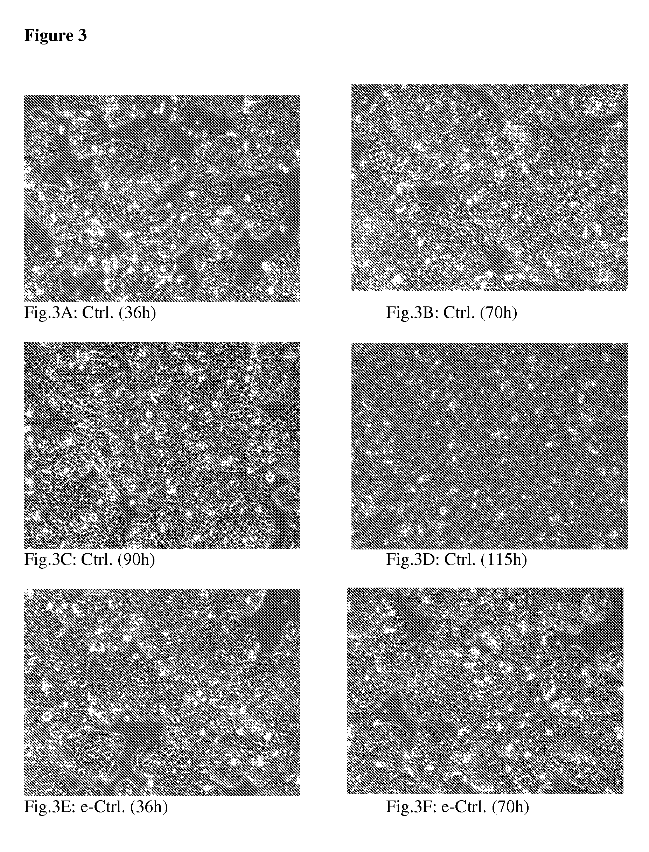

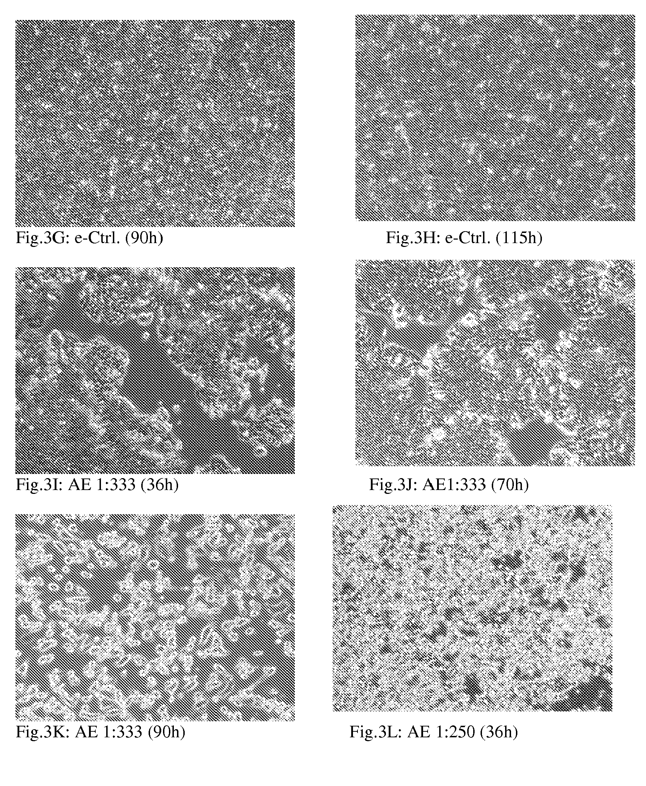

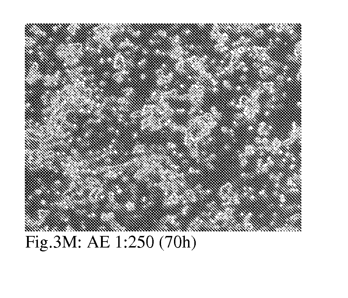

The results (see Figures 3A to 3M) show that with increasing amounts of the aqueous extract, cells display a decreasing vitality and ultimately die.

The colon cancer cell line HT229 was cultivated at 37°C, 95% humidity, 5% CO₂ for different times in standard medium comprising the organic extract (OE) of Example 3 at dilutions of 1:1000, 1:500, 1:333 and 1:250. As control, either pure cell culture medium (ctrl.) or cell culture medium with 70% alcohol at a 1:250 dilution (e-ctrl.) was used.

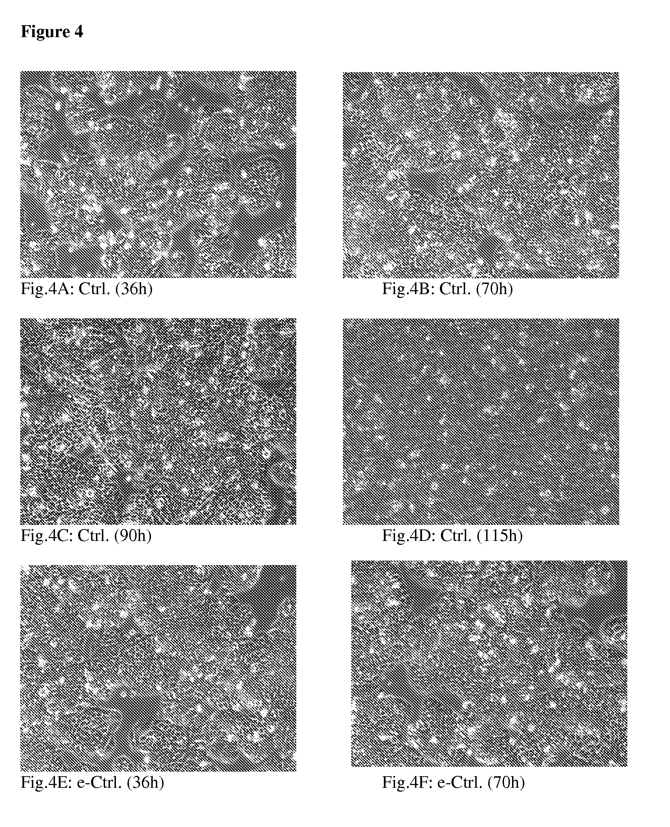

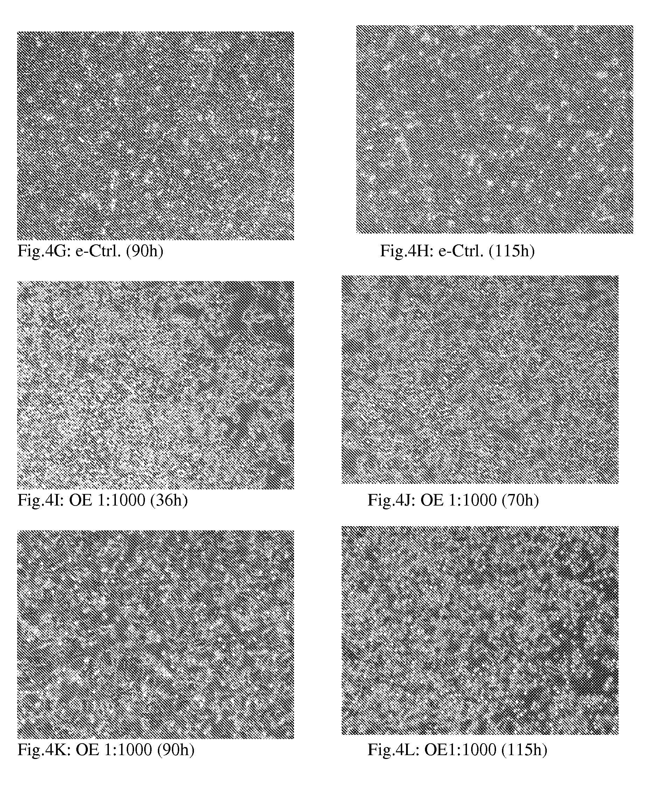

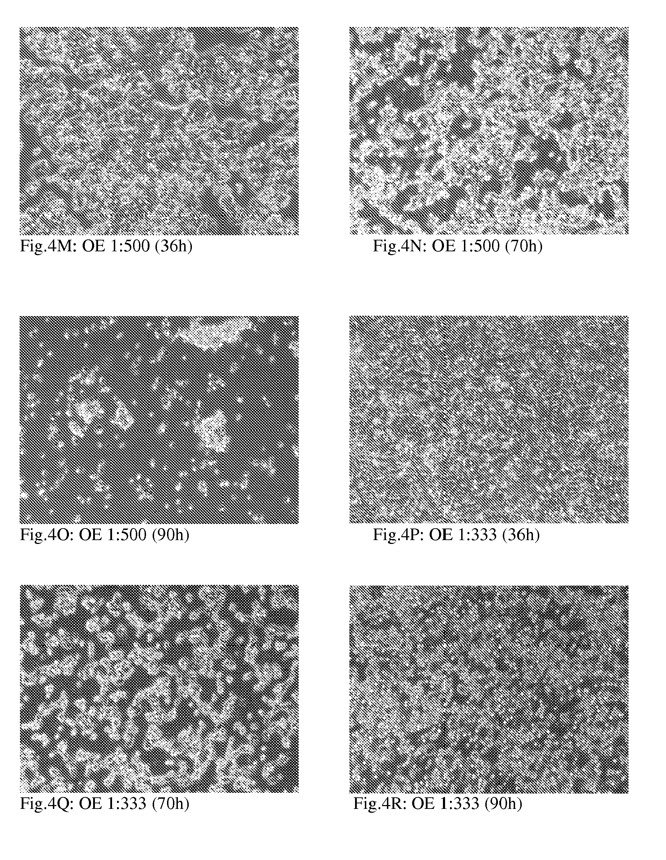

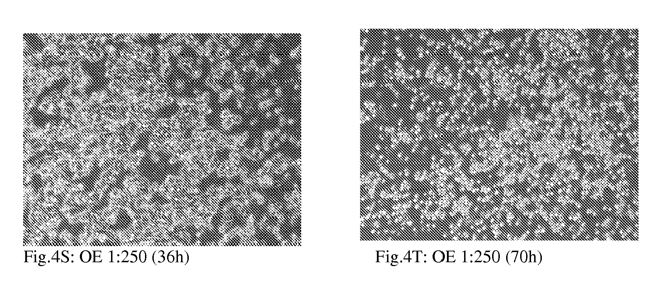

The results (see Figures 4A to 4T) show that with increasing amounts of the organic extract, cells display a decreasing vitality and ultimately die.

### Example 6 - Effects of extracts on the kidney cancer cell line CRL-1573

The kidney cancer cell line CRL-1573 was cultivated at 37°C, 95% humidity, 5% CO₂ for different times in standard medium comprising the aqueous extract (AE) of Example 1 at dilutions of 1:500, 1:333 and 1:250. As control, either pure cell culture medium (ctrl.) or cell culture medium with 70% alcohol at a 1:250 dilution (e-ctrl.) was used.

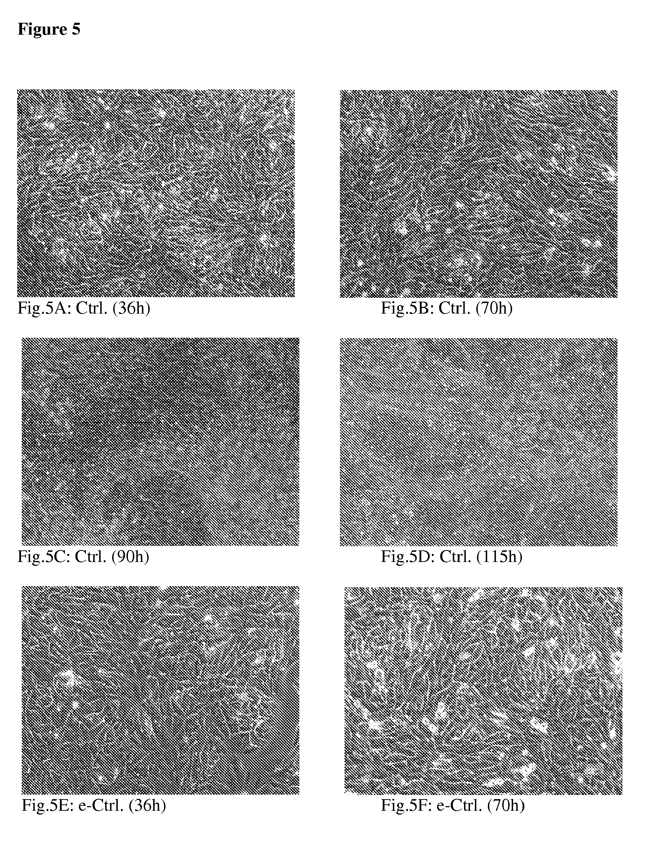

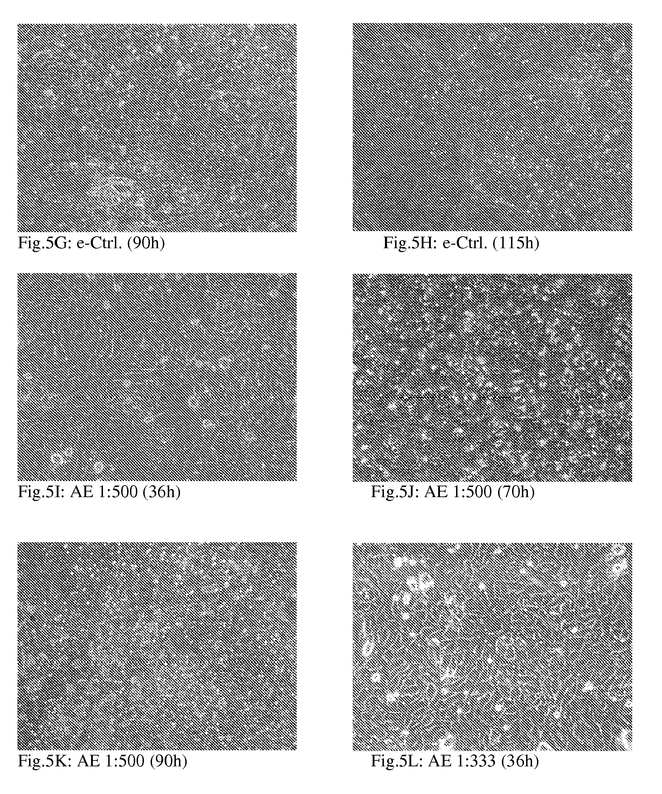

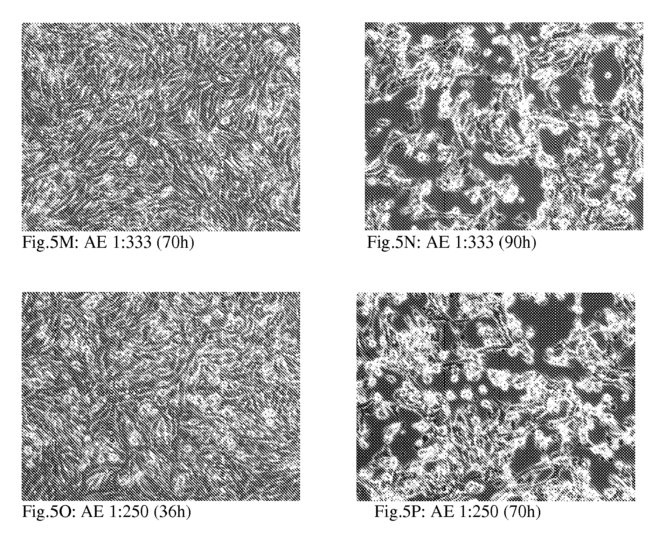

The results (see Figures 5A to 5P) show that with increasing amounts of the aqueous extract, cells display a decreasing vitality and ultimately die.

The kidney cancer cell line CRL-1573 was cultivated at 37°C, 95% humidity, 5% CO₂ for different times in standard medium comprising the organic extract (OE) of Example 3 at dilutions of 1:1000, 1:500, 1:333 and 1:250. As control, either pure cell culture medium (ctrl.) or cell culture medium with 70% alcohol at a 1:250 dilution (e-ctrl.) was used.

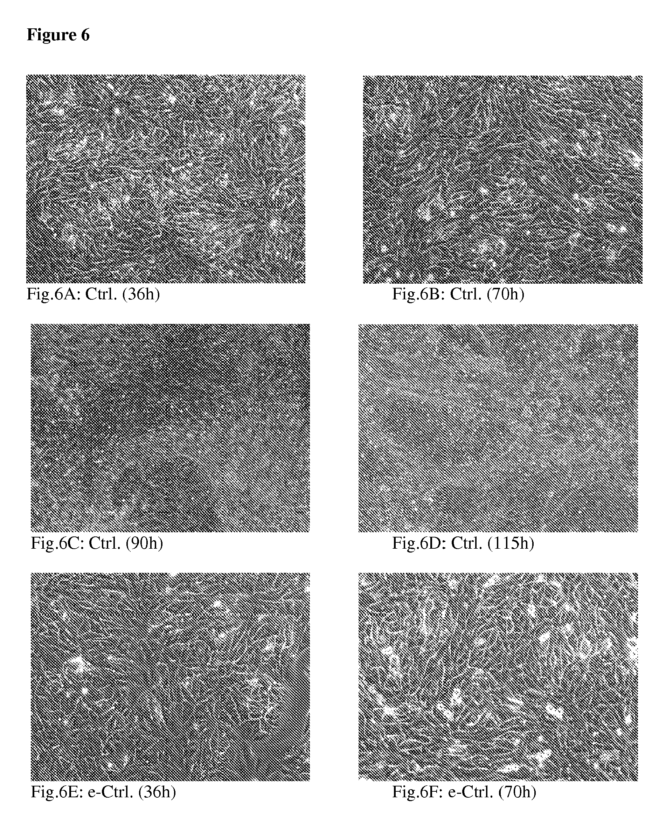

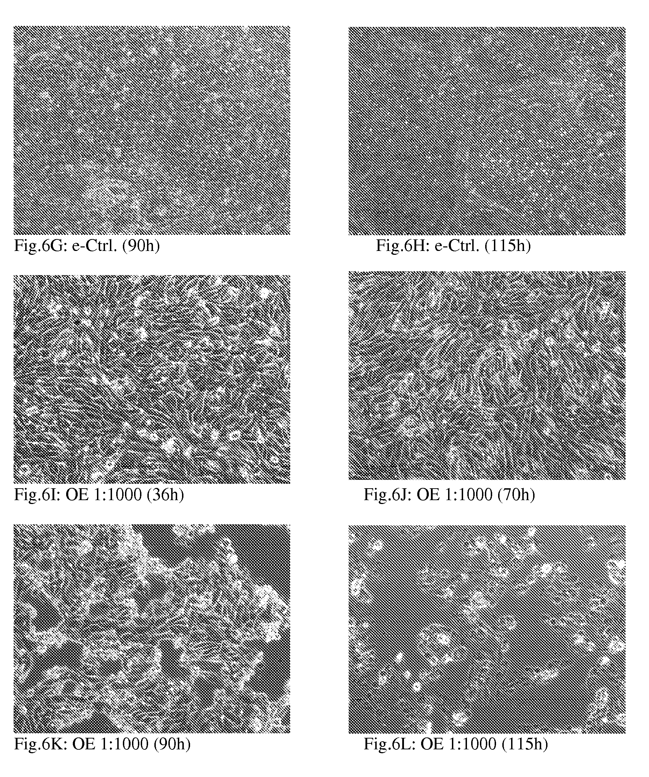

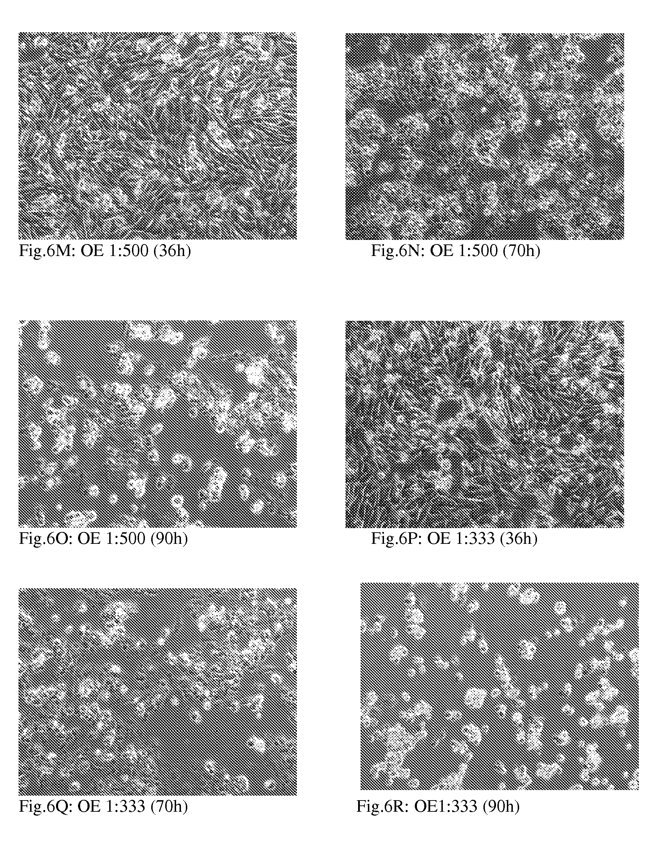

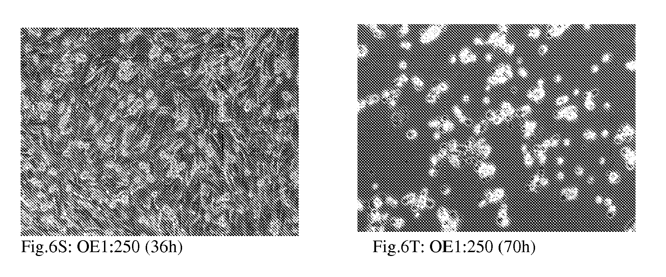

The results (see Figures 6A to 6T) show that with increasing amounts of the organic extract, cells display a decreasing vitality and ultimately die.

The kidney cancer cell line CRL-1573 was cultivated at 37°C, 95% humidity, 5% CO₂ for different times in standard medium comprising the alcoholic extract (A1E) of Example 2 at dilutions of 1:1000, 1:500, 1:333 and 1:250. As control, either pure cell culture medium (ctrl.) or cell culture medium with 70% alcohol at a 1:250 dilution (e-ctrl.) was used.

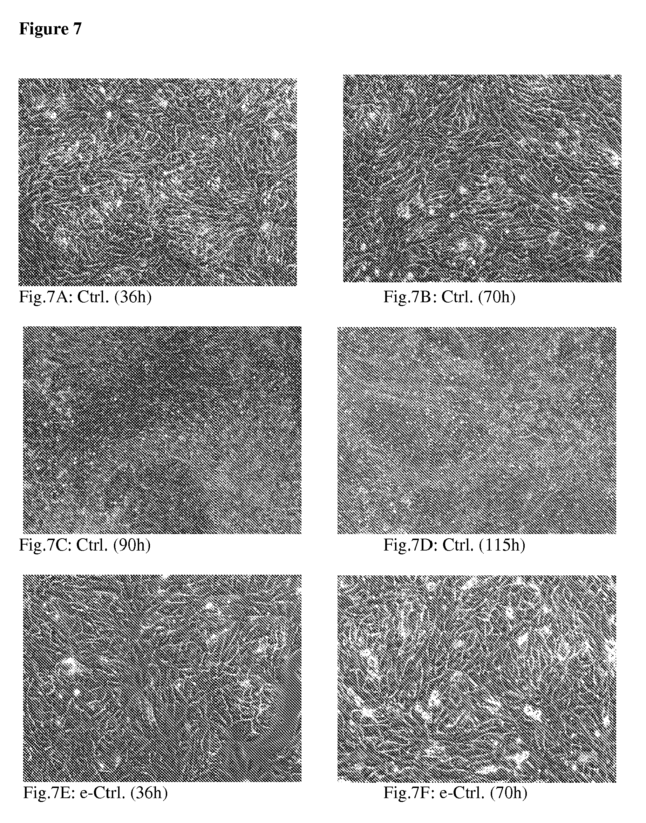

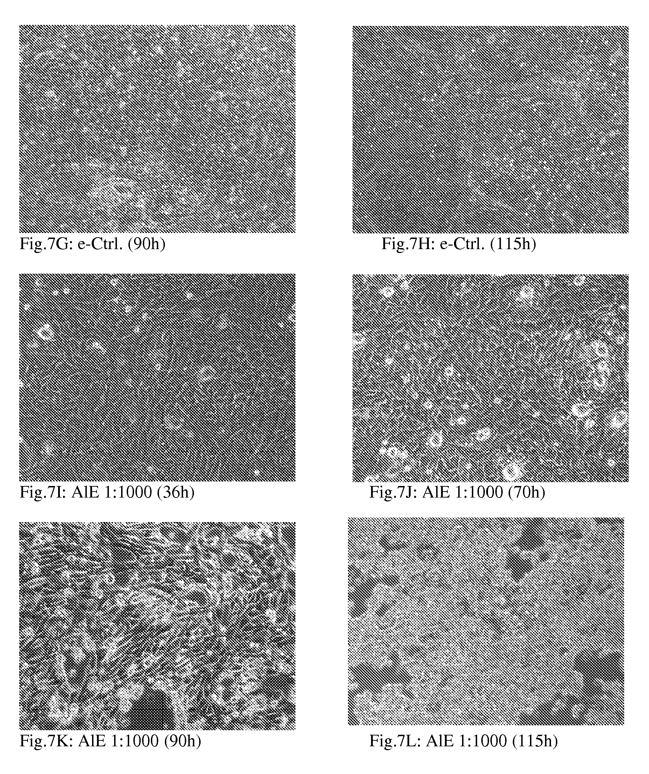

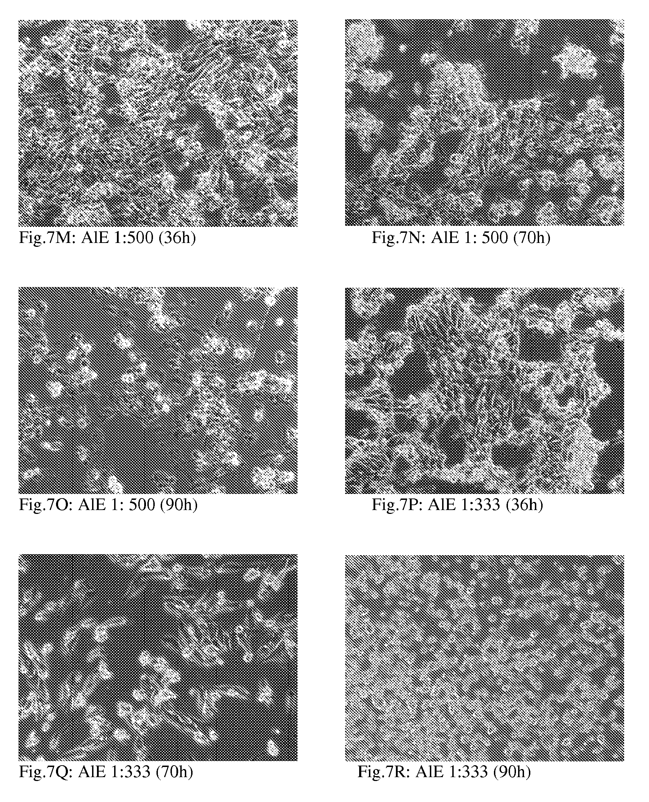

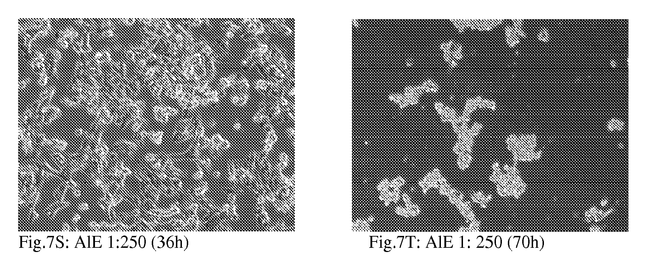

The results (see Figures 7A to 7T) show that with increasing amounts of the alcoholic extract, cells display a decreasing vitality and ultimately die.

### Example 7 - Effects of extracts on the glioblastoma cell line HT-11

The glioblastoma cancer cell line HT-11 was cultivated at 37°C, 95% humidity, 5% CO₂ for different times in standard medium comprising the aqueous extract (AE) of Example 1 at dilutions of 1:333 and 1:250. As control, either pure cell culture medium (ctrl.) or cell culture medium with 70% alcohol at a 1:250 dilution (e-ctrl.) was used.

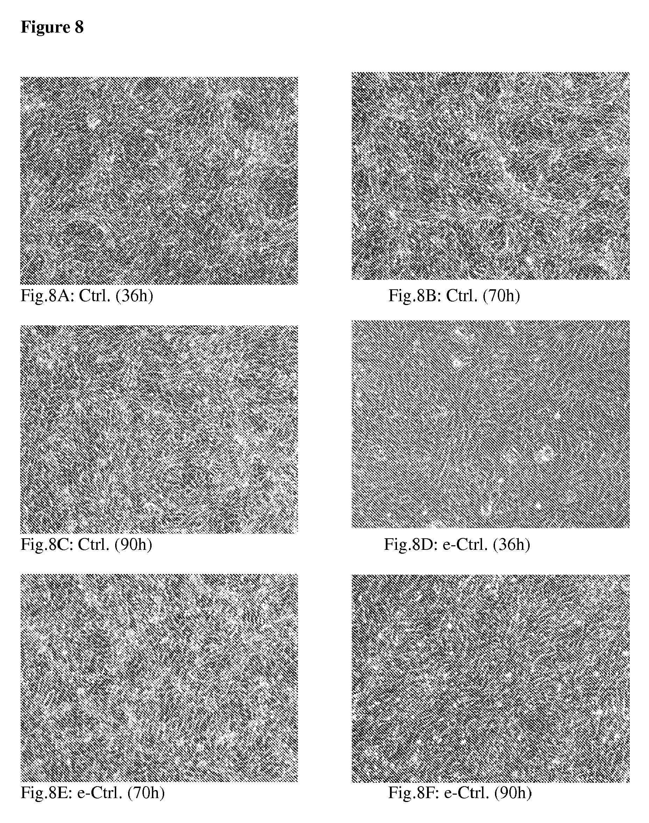

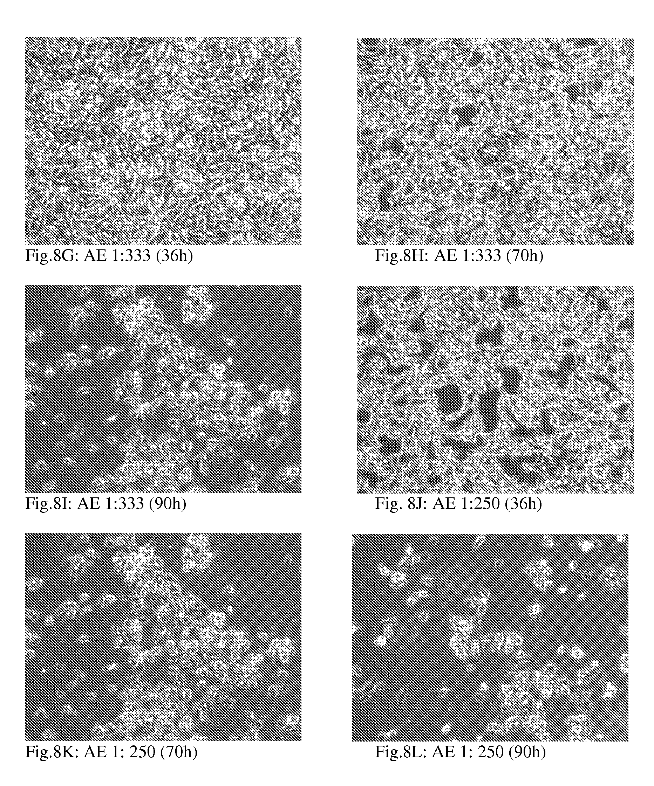

The results (see Figures 8A to 8L) show that with increasing amounts of the aqueous extract, cells display a decreasing vitality and ultimately die.

The glioblastoma cancer cell line HT-11was cultivated at 37°C, 95% humidity, 5% CO₂ for different times in standard medium comprising the organic extract (OE) of Example 3 at dilutions of 1:500, 1:333 and 1:250. As control, either pure cell culture medium (ctrl.) or cell culture medium with 70% alcohol at a 1:250 dilution (e-ctrl.) was used.

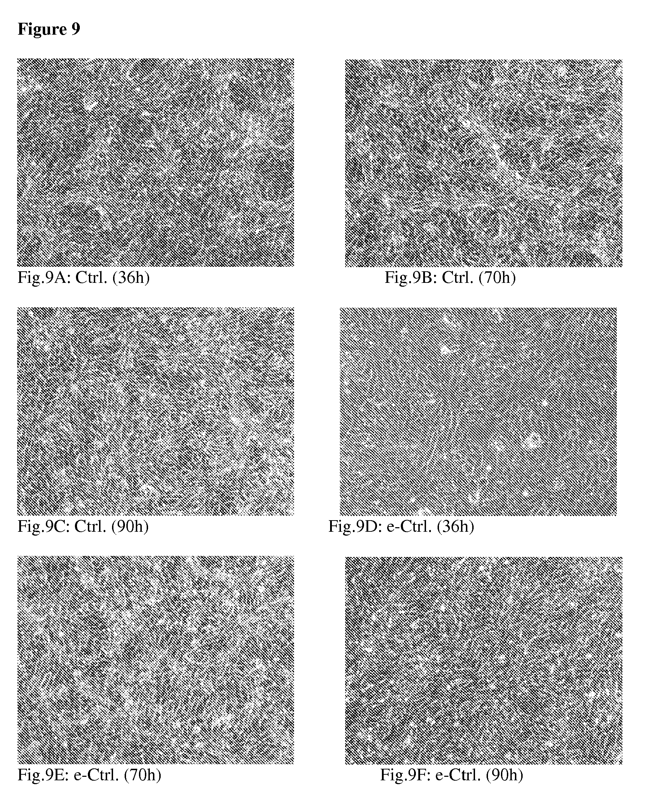

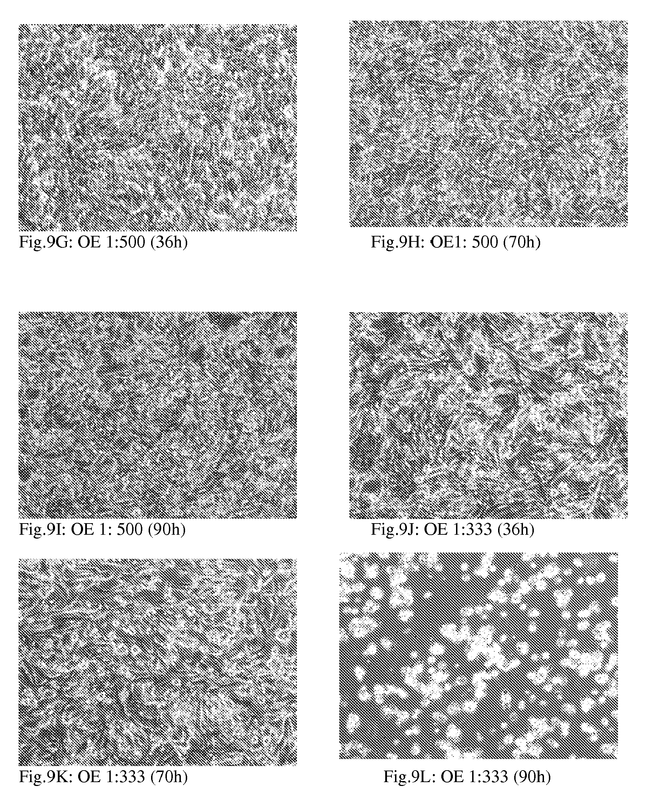

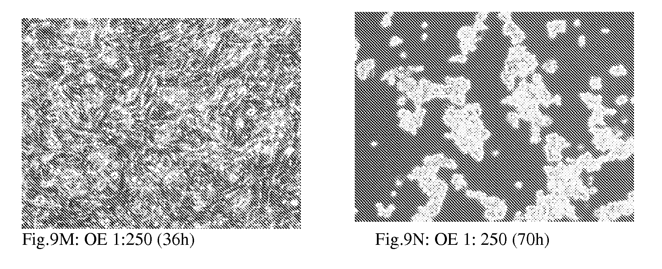

The results (see Figures 9A to 9N) show that with increasing amounts of the organic extract, cells display a decreasing vitality and ultimately die.

### Example 8 - Effects of extracts on the endothelial cell line HUVEC

The endothelial cell line HUVEC was cultivated at 37°C, 95% humidity, 5% CO₂ for different times in standard medium comprising the aqueous extract (AE) of Example 1 at dilutions of 1:333, 1:250 and 1:100. As control, either pure cell culture medium (ctrl.) or cell culture medium with 70% alcohol at a 1:100 dilution (e-ctrl.) was used.

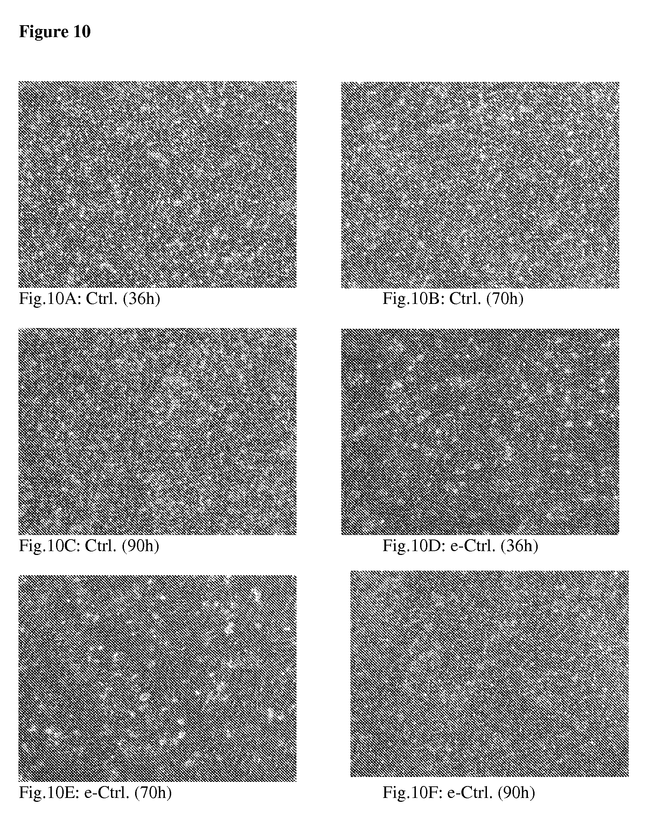

The results are shown in Figures 10A to 10P.

The endothelial cell line HUVEC was cultivated at 37°C, 95% humidity, 5% CO₂ for different times in standard medium comprising the organic extract (OE) of Example 3 at dilutions of 1:500, 1:333 and 1:250. As control, either pure cell culture medium (ctrl.) or cell culture medium with 70% alcohol at a 1:250 dilution (e-ctrl.) was used.

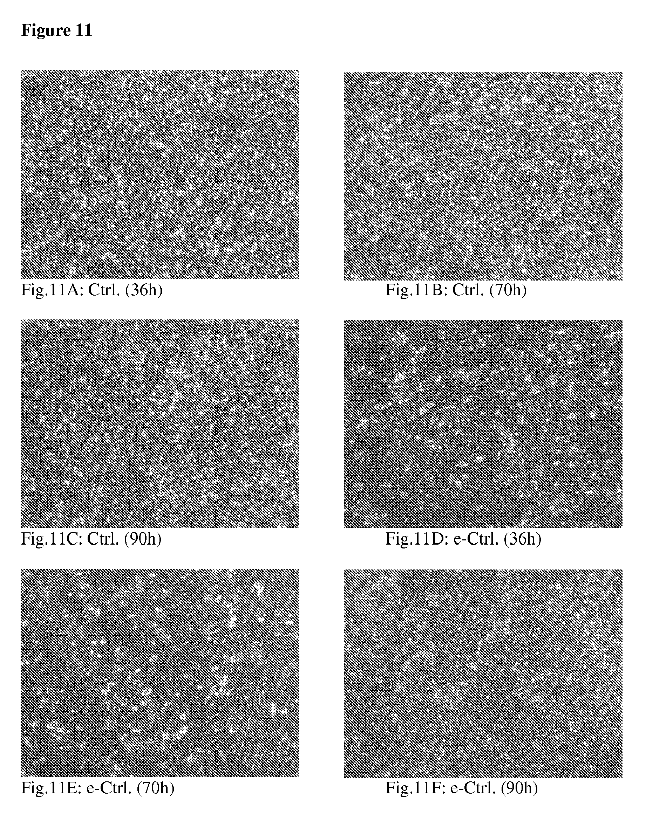

The results are shown in Figures 11A to 11P.

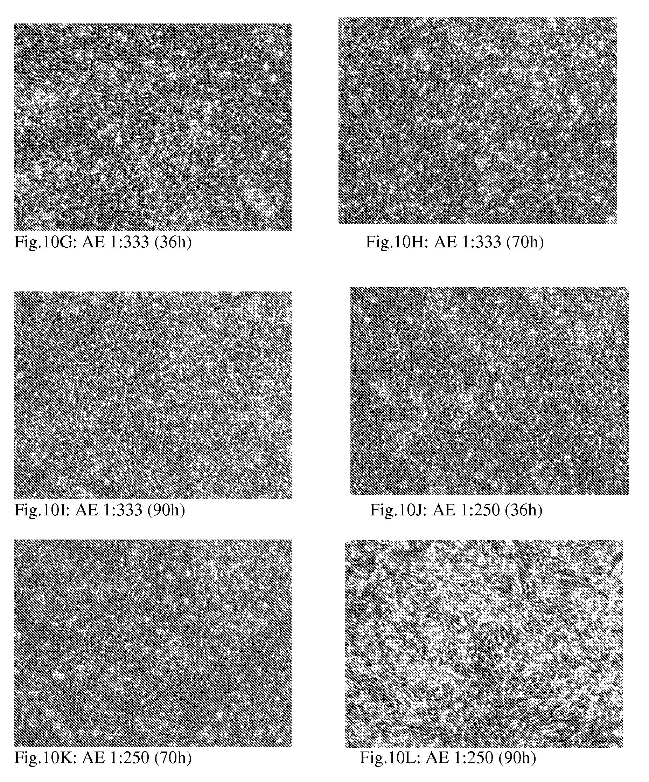

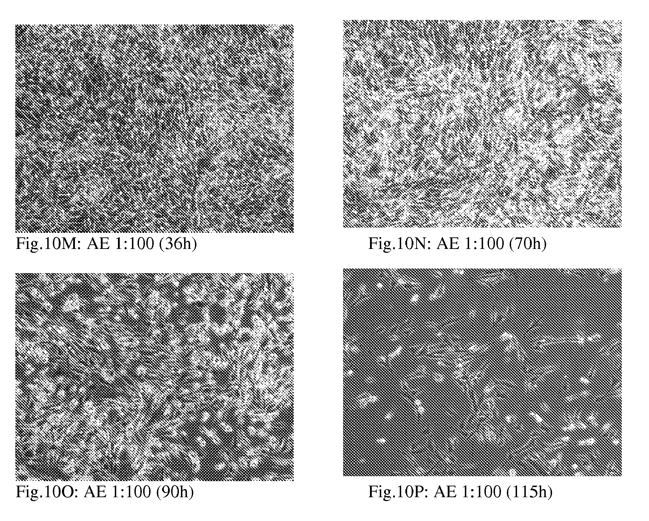

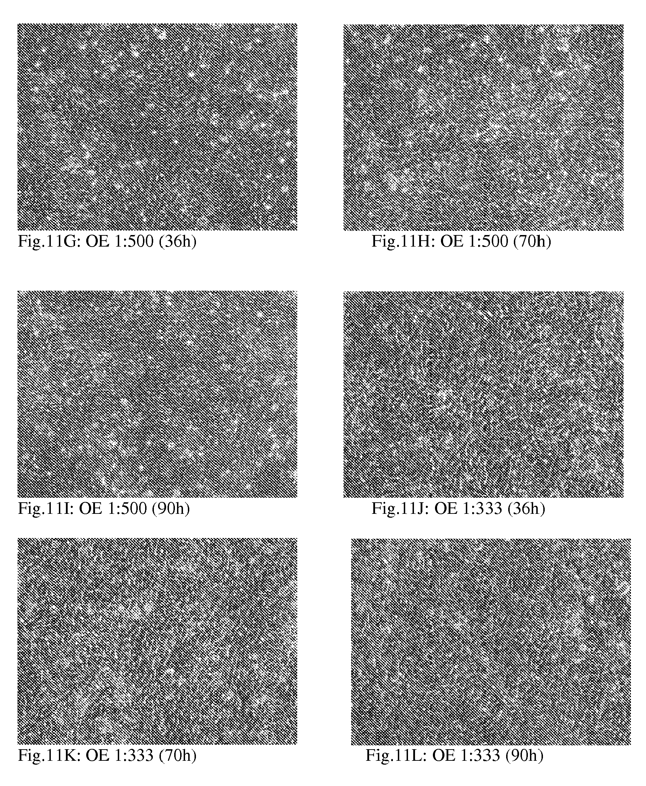

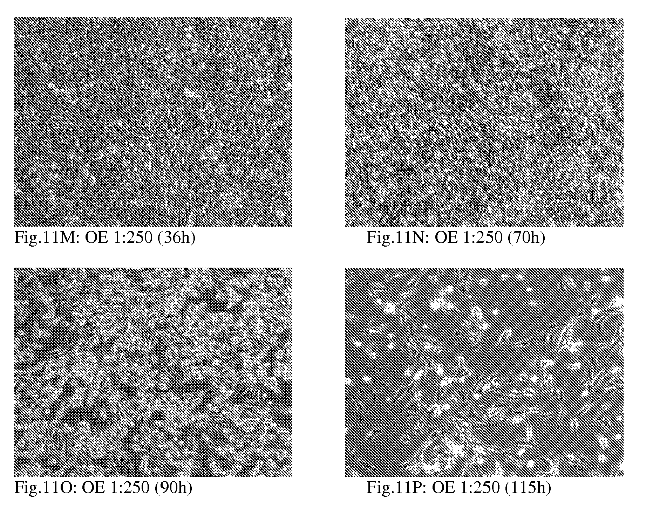

The results of Figures 10 and 11 show that with increasing amounts of the aqueous or organic extract, cells display a decreasing vitality and ultimately die. However, these effects are observed only at higher concentrations compared to the tumor cell lines of example 4 to 7.

## Claims

1. A pharmaceutical composition comprising an extract obtained from a plant of a species of the *Warburgia* genus comprising:
*Warburgia ugandensis,*
*Warburgia salutaris,*
*Warburgia stuhlmannii,*
*Warburgia elongata,* and
*Warburgia breyeri*
for use in the treatment of cancer.

2. An extract obtained from plants wherein said plants are selected from the *Canellaceae* family.

3. An extract according to claim 2, wherein the plant is a species of the *Warburgia* genus.

4. An extract according to claim 3, wherein the species is selected from the group comprising;
*Warburgia ugandensis,*
*Warburgia salutaris,*
*Warburgia stuhlmannii,*
*Warburgia elongata,* and
*Warburgia breyeri.*

5. An extract according to any of claims 2 to 4, wherein said extract is obtainable by subjecting material obtained from fruits, roots, leaves and/or bark of a *Warburgia* species to organic, aqueous or alcoholic extraction liquids.

6. An extract according to claim 5, wherein the aqueous extraction liquid is water.

7. An extract of claim 5, wherein said alcoholic extraction liquid comprises ethanol as an alcohol component which is present in an amount in the range of about 50% (v/v) to about 98% (v/v) based on the complete volume of the extraction liquid.

8. An extract of claim 7, wherein said extract is obtained by subjecting bark material of *Warburgia ugandensis* to an extraction liquid comprising ethanol as a first alcoholic component in the range of about 60% (v/v) to about 80% (v/v) such about 70(v/v)% ethanol and water as a second non-alcoholic component.

9. An extract of claim 5, wherein said organic extraction liquid comprises CH₂Cl₂, CHCl₃, toluol, tetrahydorfuran, diethylether or petroleum ether.

10. An extract of claim 9, wherein said extract is obtained by subjecting bark material of *Warburgia ugandensis* to an extraction liquid comprising CH₂Cl₂.

11. Pharmaceutical composition comprising an extract of any of claims 2 to 10 and optionally pharmaceutically acceptable excipients.

12. Pharmaceutical composition comprising an extract of any of claims 2 to 10 and optionally pharmaceutically acceptable excipients for use in the treatment of cancer.

13. Pharmaceutical composition of claim 12 for use in the treatment of cancer wherein said cancer is selected from the group of colon cancer, breast cancer, kidney cancer and/or glioblastoma.

14. Use of an extract as defined in any of claims 2 to 10 in the manufacture of a medicament for the treatment of cancer.

15. Use according to claim 14 for the treatment of colon cancer, breast cancer, kidney cancer and/or glioblastoma.
